(19) Europäisches Patentamt
European Patent Office
Office européen des brevets

(11) **EP 2 229 188 B1**

(12) **EUROPEAN PATENT SPECIFICATION**

(45) Date of publication and mention
of the grant of the patent:
**02.04.2014 Bulletin 2014/14**

(51) Int Cl.:
*A61L 15/46* (2006.01)    *A61L 15/34* (2006.01)
*A61L 15/20* (2006.01)    *A61F 13/15* (2006.01)

(21) Application number: **07852153.1**

(22) Date of filing: **21.12.2007**

(86) International application number:
**PCT/SE2007/001159**

(87) International publication number:
**WO 2009/082287 (02.07.2009 Gazette 2009/27)**

(54) **ABSORBENT ARTICLE WITH ODOUR CONTROL SUBSTANE**

SAUGFÄHIGER ARTIKEL MIT GERUCHSKONTROLLSUBSTANZ

ARTICLE ABSORBANT À SUBSTANCE DÉSODORISANTE

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**22.09.2010 Bulletin 2010/38**

(73) Proprietor: **SCA Hygiene Products AB
405 03 Göteborg (SE)**

(72) Inventors:
• **ANDREASSON, Bo
  S-852 38 Sundsvall (SE)**
• **FORSGREN BRUSK, Ulla
  S-435 43 Pixbo (SE)**
• **MALMGREN, Kent
  S-854 63 Sundsvall (SE)**
• **STRIDFELDT, Chatrine
  S-436 58 Hovås (SE)**

(74) Representative: **Valea AB
Lindholmspiren 5
417 56 Göteborg (SE)**

(56) References cited:
**EP-A2- 1 192 955    WO-A1-2007/067111
GB-A- 1 282 889    US-A- 2 356 062**

• L.A. SECHI, I. LEZCANO, N. NUNEZ, M. ESPIM, I. DUPRÈ, A. PINNA, P. MOLICOTTI, G. FADDA AND S. ZANETTI: "Antibacterial activity of ozonized sunflower oil (Oleozon)", JOURNAL OF APPLIED MICROBIOLOGY, vol. 90, 2001, pages 279-284, XP002684748,
• MARITA F. DIAZ, REBECA HERNANDEZ, GOTYBELL MARTINEZ, GENNY VIDAL, MAGALI GOMEZ, HAROLD FERNANDEZ AND RAFAEL GARCÉS: "Comparative Study of ozonized olive oil and ozonized sunflower oil", J. BRAZ.CHEM. SOC., vol. 17, no. 2, 2006, pages 403-407, XP002684749,

**Description**

*TECHNICAL FIELD*

The present invention refers to an absorbent article such as a sanitary napkin, panty liner, diaper, pant diaper, adult incontinence guard, said article comprises an absorbent core and a fluid impervious backsheet, wherein the article contains an odour control substance.

*BACKGROUND OF THE INVENTION*

Odour control has become an important factor in absorbent articles. Odours or unpleasant smells occur e.g. as a result of discharges from the wearer of an absorbent article or as a result of the storage of bodily fluids in the article. These odours can be embarrassing for the wearer of the article. It is important, therefore, to reduce or prevent odours from occurring in absorbent articles while they are being worn.

Examples of odour substances that may occur in absorbent articles are sulphur compounds, aldehydes, indoles, amines etc.

Various methods are used to prevent or reduce odours in absorbent articles that have arisen in conjunction with the discharge of bodily fluids. The methods are based on 1) masking of the odours; 2) a chemical reaction, for example in the form of neutralization, with an acid/base system; 3) adsorption/absorption of odours involving the creation of surfaces which exhibit a special affinity to the odours or large specific surfaces/cavities which are able to bind the odours concerned and thus to prevent them from remaining in gaseous form, or 4) bacteria inhibitors which reduce/control the growth of bacteria and associated odours that have arisen because of high bacteria counts.

Perfumes or fragrances are used, for example, in order to mask odours/smells. Maskers do not remove the smells and must be added in an appropriate quantity to ensure that the smell does not penetrate or that the perfume does not smell too strongly. Zeolites, silicone dioxide, clays, active carbon and/or cyclodextrin, for example, are used for the adsorption of odour substances. Some of these are susceptible to moisture, however, which restricts their effectiveness. Sodium bicarbonate, citric acid and/or superabsorbent materials with a low pH are used for the neutralization of odours. Bacteria can generate substances with an unpleasant smell, and copper acetate, a superabsorbent material with silver ions and/or an acidic superabsorbent material can be used to reduce the growth of bacteria.

The above-mentioned odour control substances are effective against different kinds of odours and act with different mechanisms.

A number of odours/smells are hydrophobic, and such smells are absorbed and/or adsorbed by hydrophobic odour control substances. Hydrophobic odouriferous substances include, for example, certain organic acids, sulphur compounds, aldehydes, indole, amines, etc., which commonly occur in conjunction with the use of absorbent articles.

Described in US 6 147 028 is an odour control substance in the form of polysiloxane-coated starch granules that are used in absorbent products. The starch granules have a hydrophobic surface, and they absorb hydrophobic material from the air.

US 6 479 150 describes material layers of thermoplastic fibers with a hydrophobic odour control substance that is modified with a surface-active substance in order to make the layer wettable. The odour control substance is, for example, an aromatic odour control substance.

GB1282889 discloses deodorant compositions comprising as active ingredient at least one calcium, aluminium, magnesium or zinc salt of an unsaturated aliphatic hydroxycarboxylic acid having at least 17, preferably 17-21, carbon atoms. Preferred unsaturated aliphatic hydroxycarboxylic acids are ricinoleic acid, ricinelaidic acid, dihydroxyoctadeconic acid, and carboxylic acids with multiple hydroxylation and single or double unsaturation obtained from the oxidation of linolenic acid.

WO2007/067111 discloses an absorbent product, such as a diaper, a sanitary napkin or an incontinence product, whereby the product comprises odour control agent(s) selected from linear dextrin, activated, amylose such as V-amylose and cyclodextrin.

US2356062 discloses compositions of the type that function by the release of nascent oxygen for sustained therapeutic action.

EP1192955 discloses a barrier agent for an absorbent article comprising an oil soluble antioxidant, wherein the antioxidant is one or more vitamins selected from the vitamin groups consisting of vitamin A group, vitamin B group, vitamin D group, vitamin E group, and vitamin K group.

Previously disclosed odour control substances suffer from the disadvantage, among other things, that they are difficult to distribute uniformly throughout the whole of the absorbent product. This is attributable to the fact that previously disclosed odour control materials often consist of solid particles, which cannot be distributed continuously over the internal and external surfaces of the product and as such reduce the degree of coverage. The possibility of trapping undesirable odours in an effective manner is reduced in this way.

The need remains to develop odour control substances for hygiene products, and one object of the present invention is to solve the above-mentioned problems and to develop an effectively functioning odour control material.

## SUMMARY OF THE INVENTION

The above defined problem is solved in the present invention by an absorbent article such as a sanitary napkin, panty liner, diaper, pant diaper, adult incontinence guard, said article comprises an absorbent core and a fluid impervious backsheet, wherein to the article at least one oxidized lipid has been added as an odour control substance.

In one aspect of the invention the lipids have been oxidized under controlled conditions. Preferably the oxidized lipids have a peroxide value as measured by AOCS Official Method Cd 8-53 of at least 20, preferably at least 30 and more preferably at least 40 meq/kg.

In one embodiment the absorbent core comprises hydrophilic fibers treated with said oxidized lipids. Preferably at least part of said hydrophilic fibers are cellulose fibers.

In a further aspect of the invention at least 0.2% by weight of the oxidized lipids has been added to said absorbent core, calculated on the total weight of the hydrophilic fibers contained in the absorbent core.

In one embodiment between 0.2 and 50% by weight, preferably between 0.5 and 40% by weight, more preferably between 1 and 35% by weight and most preferably between 3 and 30% by weight of the oxidized lipids has been added to the absorbent core, calculated on the total weight of the hydrophilic fibers contained in the absorbent core.

In one aspect of the invention the article further comprises a liquid-pervious topsheet and/or one or more additional functional layers selected from: liquid receiving layers, liquid distribution layers.

In a further aspect at least 0.2% by weight of the oxidized lipids has been added to said topsheet and/or additional functional layer, calculated on the total weight of said topsheet and/or additional functional layer.

In one embodiment between 0.2 and 50% by weight, preferably between 0.5 and 40% by weight, more preferably between 1 and 35% by weight and most preferably between 3 and 30% by weight of the oxidized lipids has been added to the topsheet and/or additional functional layer, calculated on the total weight of the topsheet and/or additional functional layers.

In embodiment of the invention said article has a longitudinal, y, and a transverse, x, direction, a pair of longitudinal edges and a pair of transverse edges, wherein the odour control substance in the form of oxidized lipids is present in higher amounts in areas close to the longitudinal and/or transverse edges of the article than in the central area of the article, intended as the fluid-receiving area.

According to a further embodiment the absorbent article comprises elastic means, for example leg elastics and/or waist elastics, wherein at least one oxidized lipid has been added to said elastic means.

According to one embodiment the lipids are fatty acids or derivatives thereof. The fatty acid derivatives are in a further embodiment esters of fatty acids, especially triglycerides.

According to a further embodiment at least part of said fatty acids and/or fatty acid derivatives are unsaturated.

In one embodiment said oxidized lipids are oxidized by treatment with ozone.

## DESCRIPTION OF THE DRAWINGS

Fig. 1 is a schematic plan view of an absorbent article in the form of a sanitary napkin or incontinence guard.
Fig. 2 is a cross section according to the line II-II in Fig. 1.

## DEFINITIONS

The term "absorbent article" refers to products that are placed against the skin of the wearer to absorb and contain body exudates, like urine, faeces and menstrual fluid. The invention mainly refers to disposable absorbent articles, which means articles that are not intended to be laundered or otherwise restored or reused as an absorbent article after use. Examples of disposable absorbent articles include feminine hygiene products such as sanitary napkins, panty liners and sanitary panties, diapers and pant diapers for infants and incontinent adults, incontinence pads, diaper inserts and the like.

The term "lipid" denotes all fat-soluble (lipophilic), naturally-occurring substances, such as fats, oils, waxes, cholesterol, steroids, monoglycerides, diglycerides, triglycerides, phospholipids, and others.

By "oxidized lipids" is meant that the lipids have undergone an oxidation process wherein oxygen has been introduced in the lipid molecular structure. The oxidation agent is any agent, which leads to oxidation of the lipid structure, e.g. oxygen gas, ozone or peroxides.

By "oxidized under controlled conditions" is meant that the substrate, i.e. the lipid has been oxidized to a degree wherein further oxidation caused by autoxidation from contact with air is substantially prevented. Preferably the lipids have been oxidized so that they have a peroxide value as measured by AOCS Official Method Cd 8-53 of at least 20 meq/kg.

## DETAILED DESCRIPTION OF THE INVENTION

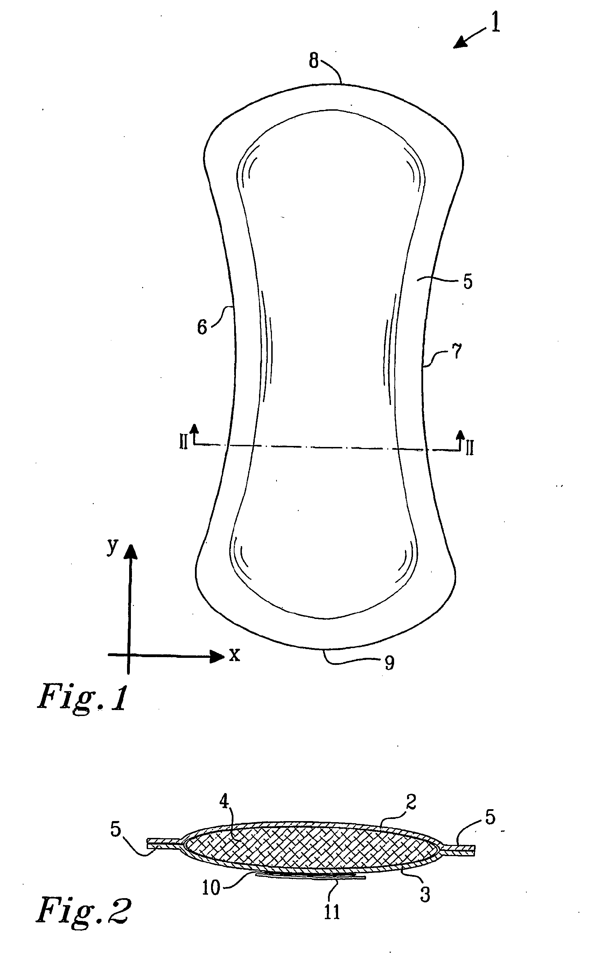

Fig. 1 and 2 show an embodiment of an absorbent article in the form of a sanitary napkin or an incontinence guard 1 intended to be worn as an insert in a pair of pants. The article 1 typically comprises a liquid permeable topsheet 2, a liquid impermeable backsheet 3 and an absorbent core 4 enclosed therebetween. The article has a longitudinal direction, y, and a transverse direction, x. It has a pair of longitudinal side edges 6 and 7 and a pair of transverse edges 8 and 9.

The liquid permeable topsheet 2 can be composed of a nonwoven material, e g spunbonded, meltblown, carded, hydroentangled, wetlaid etc. Suitable nonwoven materials can be composed of natural fibers, such as woodpulp or cotton fibres, manmade fibres, such as polyester, polyethylene, polypropylene, viscose etc. or from a mixture of natural and manmade fibres. The topsheet material may further be composed of tow fibres, which may be bonded to each other in a bonding pattern, as e.g. disclosed in EP-A-1 035 818. Further examples of topsheet materials are porous foams, apertured plastic films etc. The materials suited as topsheet materials should be soft and non-irritating to the skin and be readily penetrated by body fluid, such as urine or menstrual fluid.

The liquid impermeable backsheet 3 may consist of a thin plastic film, e.g. a polyethylene or polypropylene film, a nonwoven material coated with a liquid impervious material, a hydrophobic nonwoven material, which resists liquid penetration or laminates of plastic films and nonwoven materials. The backsheet material may be breathable so as to allow vapour to escape from the absorbent core, while still preventing liquids from passing through the backsheet material.

The topsheet 2 and the backsheet material 3 have a somewhat greater extension in the plane than the absorbent core 4 and extend outside the edges thereof. The layers 2 and 3 are connected to each other within the projecting portions 5 thereof, e g by gluing or welding by heat or ultrasonic. The topsheet and/or the backsheet may further be attached to the absorbent core by any method known in the art, such as adhesive or welding by heat or ultrasonic etc. The absorbent core may also be unattached to the topsheet and/or the backsheet.

A fastening means in the form of a region 10 of an adhesive is provided on the side of the backsheet facing away from the wearer in use. The adhesive may releasably attach to the undergarment of the wearer. A release paper 11 protects the adhesive region before use. The adhesive region 10 may have any suitable configuration, such as elongate or transverse strips, dots, full-coated areas etc.

In other embodiments of absorbent articles according to the invention other types of fasteners, like friction fasteners, tape tabs or mechanical fasteners like hook-and-loop fasteners etc may be used to fasten the articles to the underwear or around the waist of the wearer. Some absorbent articles are in the form of pants and therefore do not need special fastening means. In other cases the absorbent article is worn in special elastic pants without the need for additional fasteners.

The absorbent core 4 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent core. It is also common to have absorbent bodies comprising layers of different material with different properties with respect to liquid acquisition capacity, liquid distribution capacity and storage capacity. This is well-known to the person skilled in the art and does therefore not have to be described in detail. The thin absorbent bodies, which are common in today's absorbent articles, often comprise a compressed mixed or layered structure of cellulosic fluff pulp and superabsorbent material. The size and absorbent capacity of the absorbent core may be varied to be suited for different uses such as sanitary napkins, pantiliners, adult incontinence pads and diapers, baby diapers, pant diapers, etc.

It is understood that the absorbent article described above and shown in the drawings only represents one non-limiting example and that the present invention is not limited thereto, but can be used in any type of absorbent articles as defined above.

The object of the present invention is to provide absorbent articles such as sanitary napkins, panty-liners, diapers, incontinence guards suitable for absorbing bodily fluids and which contain an odour control substance which effectively prevents or reduces odours in such articles that have arisen in conjunction with the discharge of bodily fluids.

It has according to the invention been shown that oxidized lipids, are very effective in reducing certain odouriferous substances which are commonly occurring in absorbent articles. Natural animal-derived or plant-derived lipids are very often mixtures of mono-, di- and triglycerides and free fatty acids. The lipids can be purified, hydrated, refined, modified and used individually or in different mixtures. Examples of suitable lipids which originate from animals can be found in bees waxes, emu oil, lactis lipida, lanolin, shark's liver oil, lard, whale oil, butter fat and tallow. Examples of suitable lipids which originate from plants can be found in apricot kernel oil, ground nut oil, avocado oil/wax, blackcurrant seed oil, borage seed oil, Brazil nut oil, castor oil, cocoa butter, coconut oil, maize oil, cotton seed oil, rose hip seed oil, evening primrose oil, grape seed oil, linseed oil, mango seed oil, rose oil, olive oil, orange wax, palm oil, ground nut oil, rice wax, sesame seed oil, shea butter, soybean oil, sunflower seed wax, peanut oil, sesame oil, safflower oil, tobaccoseed oil, poppyseed oil, teased oil, kapok oil, rice bran oil, sorghum oil, crambe oil, linseed oil, perilla oil, hempseed oil, tung oil, oiticica oil, palm kern oil, sweet almond oil and wheat germ oil. Further examples of lipids are waxy oils, which are esters of mono-alcohols, for example Jojoba oil, phospholipids etc.

Triglycerides are commonly occurring in many natural fats and oils, such as rapeseed oil, olive oil, maize oil, sunflower oil, palm oil, cocoanut oil and butter, palm oil, cacao butter, theobroma oil etc. Most of the naturally occurring triglycerides contain a mixture of saturated and unsaturated fatty acids, while the proportion of saturated and unsaturated fatty acids varies between the different oils. This proportion is usually given as the quotient: unsaturated/saturated. The unsaturated fatty acids may either be monounsaturated or polyunsaturated. The most commonly occurring fatty acids in triglycerides are palmitic acid, a saturated fatty acid, oleic acid, a monounsaturated fatty acid, linoleic and linolenic acids, which are polyunsaturated fatty acids.

The composition of some common natural oils are given in Table 1 below, which is taken from Bailey's Industrial Oil and Fat products, vol.1, editor: Daniel Swern, John Wiley & Sons Inc., New York, 1979.

Table 1

| Vegetable oil | Saturated fatty acids (weight-%) | Unsaturated fatty acids (weight-%) |
|---|---|---|
| Olive oil | 9.3-18.8 | 81.1-89.0 |
| Sunflower oil | 8.7-14.2 | 85-91 |
| Rapeseed oil | 6.2-9.5 | 90.5-93.8 |
| Maize oil | 12-18 | 82-88 |
| Cocoa butter | 59.8 | 40.2 |

Such oils and fats normally contain antioxidants, either naturally occurring or added by a supplier, so that autoxidation caused by contact with air is substantially prevented or delayed.

The lipids used in the present invention are oxidized by an oxidizing agent. Examples of useful oxidizing agents are: ozone, peroxides, oxygen gas, peroxy acids and nitrogen dioxide. For lipids containing antioxidants more powerful oxidizing agents like ozone and peroxides are required, but for lipids without any significant amounts of antioxidants, oxygen or air, i.e. autoxidation under a sufficient time period, may be sufficient.

The reactivity of different lipids is dependant on the number of double bonds, i.e. the degree of unsaturation. Saturated lipids oxidize very slowly while lipids with a high degree of unsaturation oxidize more rapidly. The relative rates of autoxidation at a temperature of 100°C of some fatty acids (not treated with antioxidants) are found in Table 2 below and are taken from the same reference as for Table 1.

**Table 2**

| Fatty acid | Chemical formula | Relative rate of oxidation |
|---|---|---|
| Stearic acid | $C_{17}H_{35}COOH$ | 0.6 |
| Oleic acid | $C_{17}H_{33}COOH$ | 6 |
| Linoleic acid | $C_{17}H_{31}COOH$ | 64 |
| Linolenic acid | $C_{17}H_{29}COOH$ | 100 |

The oxidation should preferably be performed under controlled conditions, so that after the oxidation process autoxidation is substantially prevented. Preferably the oxidized lipids should have a peroxide value as measured by AOCS Official Method Cd 8-53 of at least 20, preferably at least 30 and more preferably at least 40 meq/kg.

The lipids may be oxidized by any suitable method and by any suitable oxidation agent, for example by ozone, mixtures of ozone/air or ozone/oxygen.

At the oxidation process a series of peroxidic products may be formed, such as hydroperoxides, ozonides, diperoxides, peroxides and polyperoxides. Certain by-products may also be formed, for example ketones and aldehydes, which are less desired. These by-products may be removed by washing the lipids with a solvent after the oxidation process. Alternatively volatile undesired substances may be moved by evaporation, for example under vacuum.

It has according to the invention been shown that pulp treated with oxidized lipids, especially ozonized triglycerides, have a significant ability to reduce the emission of undesired odour compounds that are frequently occurring in absorbent articles absorbing body liquids like urine and menstrual fluid. Examples of such odour compounds are dimethyl sulfide (DMS), dimethyl disulfide (DMDS) and isovaleric aldehyde (IVA).

The amount of oxidized lipids added should be at least 0.2% of the total weight of the treated pulp.

The treated pulp may be mixed with untreated pulp and/or with superabsorbent material to form an absorbent core 4. An absorbent core can contain between 0.2 and 50% by weight, preferably between 0.5 and 40% by weight, more preferably between 1 and 35% by weight and most preferably between 3 and 30% by weight of added oxidized lipids calculated on the total weight of the hydrophilic fibers, for example pulp fibers, contained in the absorbent core.

In certain areas, for example along the edges of the absorbent core, a higher proportion of the oxidized lipids may be used than in the central area of the absorbent core. The fibres in the edge areas may then be more or less saturated with the oxidized lipids and function as leak barriers.

Different types of lipids affect the absorption properties of the pulp to a higher or smaller degree. For example triolein, which is the triglyceride of mono-unsaturated fatty acid oleic acid, did not decrease the liquid uptake of treated pulp to any higher degree, while the same amount of tristearin, which is the triglyceride of the saturated fatty acid stearic acid, resulted in a clear hydrophobization of the treated pulp. Accordingly the amount of added lipid to the pulp may vary depending on the lipids used.

The oxidized lipids may thus be added to the pulp, usually cellulosic fluff pulp, used in the absorbent core 4 of an absorbent article. Alternatively or in addition they may be added to the topsheet 2 or any additional functional layer contained in the absorbent article, such as liquid receiving layer, liquid distribution layer, liquid storage layer etc.

At least a part of the treated pulp fibers in the core can be distributed only to certain areas in the absorbent core 4. For example the treated pulp fibers are distributed or positioned in areas in the form of spaced apart islands. The proportion by weight of the hydrophobic odour control material in the aforementioned islands in the core can be 25-35 % by weight, calculated in relation to the total weight of the hydrophilic fibrous material in the core in the areas or the islands. In such areas, the proportion by weight of oxidized lipids can be higher compared with the situation in which the oxidized lipids are uniformly distributed in a core, since a greater proportion by weight of liquid-absorbent fibers is present in adjacent parts of the liquid-absorbent core in order to compensate for impaired liquid absorption by the treated pulp fibers in the areas. The treated fibers can be mixed with other fibers and/or with superabsorbent material in different proportions. The proportion of oxidized lipids should not be too high in the wetting area, because they may impair the absorption capacity of body liquid. The wetting area is located essentially in the central crotch part of the absorbent article.

At least a part of the oxidized lipids can be contained in areas along the longitudinal lateral edges of the liquid-absorbent core 4 and/or other functional layer. The odours can be taken up by the oxidized lipids when the odours are on their way out through the edges of the absorbent article. The odours can move towards the lateral edges for a variety of reasons. They can be caused to evaporate, so that they move towards the edges, the wearer of the product may move, so that ventilation occurs because of the movement.

The proportion by weight of oxidized lipids in the areas along the longitudinal lateral edges can be relatively high, since the fluid-absorption capacity can be allowed to be lower at the edges. It is also advantageous to have the hydrophobic oxidized lipids along the lateral edges of the article, since they can then function as liquid barriers and as such reduce the risk of leakage.

In other embodiments the treated fibers may be arranged in the absorbent core 4 in areas extending as longitudinal strips along the article.

In garment-like article, such as diapers and pant article the treated fibers can be positioned in a waistband region or in areas around the leg openings. They can then prevent odours from escaping from the article. Alternatively the oxidized lipids can be added to elastic means forming part of for example waist elastics or leg elastics in an absorbent article. The addition of hydrophobic substance in the form of a skincare composition is described in WO 2007/073270. The oxidized lipids according to the present invention may be incorporated in elastic means in a similar way.

In the same manner as in the absorbent core 4, the oxidized lipids can be uniformly distributed in any other layer in the absorbent article, such as the topsheet, a liquid receiving layer, a liquid distribution layer or the like. The proportion by weight of the oxidized liquid in these layers can be the same as for the absorbent core. The oxidized lipids can also be distributed in certain areas of these layers in the same way as described with respect to the absorbent core.

Depending on the kind of absorbent article product, the proportion of oxidized lipids will vary. Panty liners, for example, do not require the same quantity of odour control material as an incontinence product.

Odours in absorbent articles differ in respect of their character. In order to achieve an even better odour-controlling effect, other types of odour control materials or odour control substances can also be used in the absorbent articles according to the present invention. These can be acidified cellulose fibers, for example, and/or superabsorbent materials with a low pH. Cellulose fibers can be acidified, for example, by the addition of a buffer/acid. The acidic odour control materials deal with odoriferous substances that are alkaline, for example, such as amines and ammonia. Acidic odour control materials are capable, if added in a sufficient quantity, of lowering the pH and, by so doing, of inhibiting the growth/activity of bacteria which in turn produce substances that are able to contribute to a bad smell.

Other odour control substances can also be added to the article, for example chitosan, starch-based odour control substances and esters. The esters can be selected from among cyclical esters or esters selected from among isomentyl acetate, isomentyl propionate, isomentyl isobutyrate, isomentyl crotonate and isomentyl butyrate.

The oxidized lipids can be added to the pulp fibers, or other fibers, in conjunction with the production of the fibers or be added in the production apparatus in which the absorbent articles are produced. The lipids may either be oxidized before being added to the fibers or after addition. In the latter case the fibers, for example pulp fibers, are treated with the lipids and the treated pulp is then reacted with the oxidizing agent, for example ozone. The ozone may then at the same time act as a bleaching agent for the pulp.

**EXAMPLES**

**Ozonization of oil/fat**

The ozone was generated in an Argenotox ozone generator, type GL, Hamburg, operated at a voltage of 150V an inlet oxygen flow of 63 l/h. 200 g of each tested oil/fat was treated during a time period of 2h with an ozone/oxygen flow of 0.061 g/min. The ozone concentration of the added gas was 58 g/m$^3$.

For the more strongly ozonized sunflower oil according to table 7, having a peroxide value of 276.9 meq./kg, ozone was bubbled through 50 g oil for 5.5 h.

The gas was bubbled through the oil which was contained in a vented vessel. A magnetic stirrer was used in the vessel. The solid fats were gently heated above melting temperature, after which the gas was bubbled through the liquid fats.

The tested oils/fats are those stated in Table 3 below.

**Table 3**

|  | Saturated | Single saturated | Multiple saturated | Quotient unsaturated/saturated |
|---|---|---|---|---|
| Sunflower oil | 11 | 28 | 56 | 7,64 |
| Olive oil | 18 | 70 | 12 | 4,56 |
| Rapeseed oil | 7 | 62 | 31 | 13,29 |
| Maize oil | 12 | 28 | 55 | 6,92 |
| Cacao butter | 61 | 36 | 3 | 0,64 |

The degree of oxidation was tested by determining the peroxide value according to the test method AOCS Official Method Cd 8-53 Surplus 2003. The peroxide value for both the starting oils/fats and the ozonized oils/fats was determined. The results are given in Table 4 below.

**Table 4**

| Oil | Peroxide value (meq/kg) |
|---|---|
| Rapeseed oil | 3.82 |
| Ozonized rapeseed oil | 42.09 |
| Maize oil | 4.21 |
| Ozonized maize oil | 60.04 |
| Olive oil | 7.99 |
| Ozonized olive oil | 61.41 |
| Sunflower oil | 7.10 |
| Ozonized sunflower oil | 65.49 |
| Cacao butter | 3.32 |
| Ozonized cacao butter | 69.51 |

*Treatment of pulp with oils/fats*

Sheets of sulfate pulp from Weyerhaeuser Inc., with the designation NB416, were impregnated with a solution of the tested oil/fat in hexane. The solution contained equal amounts of hexane and oil/fat. The solution was equally distributed over the surface of the sheets. When the hexane had evaporated the sheets contained 30% by weight oil/fat and 70% by weight pulp fibers. The treated sheets were defibrated in a Braun multimixer MX32 to produce fluff pulp.

*Analysis of odour reduction*

1g of treated pulp was put in a 60 ml vial, after which 3.9 ml of 0.01 M phosphate buffered saline solution pH 7.4 from Sigma was added. Then 0.1 ml PEG300 with DMS (dimethyl sulfide), DMDS (dimethyl disulfide) and IVA (isovaleric aldehyde) was added so that the total amount of all three odour substances was 1000 ng/ml of each substance.
After 3h at 35°C a SPME fiber (Supelco), 75µm Carboxen - PDMS, was injected into the headspace above the pulp and after an additional 0.5h the SPME fiber was analyzed with gas chromatography (GC), Thermo Finnigan Trace, with a MS detector. The peak area of each odour substance was determined for samples with treated pulp and the untreated reference pulp. The GC settings were:

Temperature program for GC: 30°C (7 min), 3°C/min - 70°C (0 min), 40°C/min -250°C (7 min).
Column:ZB-624 (Zebron), 30m, 0.25 mm i.d. 1.40 µm film thickness
Inlet temperature: 250°C
Transfer line: 220°C
Mode: Splitless
MS: SIM (single ion monitoring). When DMS, IVA and DMDS were analyzed the following mass numbers were detected: 45, 46, 47, 57, 58, 61, 62, 79, 86 and 94.

*Results of odour reduction*

The tests showed that the ozonized oils/fats had a significantly higher reduction effect on the odour substances than the corresponding oils/fats that had not been ozonized. The odour reduction results are given in Table 5 below. The odour reduction was determined by comparing the peak area of the tested sample with the same peak area achieved when testing the untreated reference pulp. The calculation of the odour reduction in percent was made by the equation:

$$\text{Odour reduction} = 100 \times (1 - \text{Actual peak area}/\text{Peak area of sample with untreated pulp}) \quad [1]$$

**Table 5**

| Reduction of odour substances in % | | | |
|---|---|---|---|
| | **DMS** | **IVA** | **DMDS** |
| Sunflower oil | 0 | 0 | 31.6 |
| Ozonized sunflower oil | 99.9 | 96.7 | 99.1 |
| Cacao butter | 35.1 | 0 | 48.2 |
| Ozonized cacao butter | 79.8 | 50.0 | 59.1 |
| Rapeseed oil | 35.8 | 38.5 | 36.5 |
| Ozonized rapeseed oil | 99.4 | 96.2 | 91.2 |
| Maize oil | 87.1 | 66.0 | 88.4 |
| Ozonized maize oil | 99.9 | 96.4 | 99.6 |
| Olive oil | 84.0 | 69.2 | 73.0 |
| Ozonized olive oil | 99.9 | 97.7 | 95.9 |

*Tests with different amounts of added oils having different peroxide values*

Tests were performed with treated pulp to which had been added different amounts of ozonized sunflower oil, 0, 3, 10 and 30% by weight respectively. Two different ozonized sunflower oils were used, one having a peroxide value of 65.6 meq./kg and the other a peroxide value of 276.9 meq/kg.
The pulp was treated in the following manner:

A sheet of bleached kraft pulp with the trade name NB416 produced by the Weyerhaeuser Company was treated with oil dissolved in a suitable evaporable solvent. The solution was poured onto 10 g of the sheet, which absorbed the liquid and distributed the oil well in the fibre network. The solvent was then evaporated by simply keeping the sheets a room temperature for at least 3 h. The following solutions were prepared:

a. 0.31 g ozonised sunflower oil with a peroxide value of 65.5 dissolved in 8.27 g hexane. This addition means that the pulp sheet will contain 3 % oil.
b. 1.11 g ozonised sunflower oil with a peroxide value of 65.5 dissolved in 7,47 g hexane. This addition means that the pulp sheet will contain 10 % oil.
c. 4.29 g ozonised sunflower oil with a peroxide value of 65.5 dissolved in 4.29 g hexane. This addition means that the pulp sheet will contain 30 % oil.
d. 0.31 g ozonised sunflower oil with a peroxide value of 276.9 dissolved in 8.27 g acetone. This addition means that the pulp sheet will contain 3 % oil.
e. 1.11 g ozonised sunflower oil with a peroxide value of 276.9 dissolved in 7,47 g acetone. This addition means that the pulp sheet will contain 10 % oil.
f. 4.29 g ozonised sunflower oil with a peroxide value of 276.9 dissolved in 4.29 g acetone. This addition means that the pulp sheet will contain 30 % oil.

After evaporation of the solvent, the oil-impregnated sheets were torn into pieces and dry defibrated in a Braun multimixer MX32. The defibration was performed at maximum intensity until a fairly homogeneous fluffed pulp was formed.
For comparison, a sheet of untreated bleached kraft pulp (NB416) was defibrated in the same way.
Used chemicals:

| Sunflower oil: | Food grade oil delivered by a local provision-shop (Konsum) |
| Hexane: | Pro Analysi, from Merck |
| Acetone: | Puriss, delivered by Fluka |

The tests were then performed in the same manner as described above. The results from are shown in Table 6 and 7 below.

**Table 6**

| Reduction of odour substances in % by the addition of ozonized sunflower oil having a peroxide value of 65.6 meq./kg | | | |
|---|---|---|---|
| Addition of ozonized sunflower oil (wt%), | DMS | DMDS | IVA |
| 0 | 0 | 0 | 0 |
| 3 | 50.4 | 21.7 | 0 |
| 10 | 95.8 | 73.3 | 73.3 |
| 30 | 98.7 | 93.5 | 86.7 |

**Table 7**

| Reduction of odour substances in % by the addition of ozonized sunflower oil having a peroxide value of 276.9 meq./kg | | | |
|---|---|---|---|
| Addition of ozonized sunflower oil (wt%), | DMS | DMDS | IVA |
| 0 | 0 | 0 | 0 |
| 3 | 94.6 | 55.6 | 92.8 |
| 10 | 100.0 | 95.2 | 92.9 |
| 30 | 100.0 | 100.0 | 96.0 |

These result show that such a low addition as 3 weight% of ozonized sunflower oil can give a strong reduction of the added odour substances and that the oil having the higher peroxide value gives a stronger odour reduction. It can be mentioned that ozonized sunflower oils having peroxide values above 1000 meq./kg are known in literature. Therefore it can be assumed that an addition of much less than 3 weight% of an oil having a high peroxide value can give an acceptable odour inhibition.

*Practical odour test*

A practical sensory odour test was also performed in which the test persons smelled at the samples after the GC tests. The following results were obtained:

| Sample | Smell |
|---|---|
| Reference: | Very strong, unpleasant odour |
| 3 % ozonised sunflower oil, peroxide value 65.5 | Clear odour, reduced compared to the reference. |
| 10 % ozonised sunflower oil, peroxide value 65.5 | Weak odour |
| 30 % ozonised sunflower oil, peroxide value 65.5 | No unpleasant odour |
| 3 % ozonised sunflower oil, peroxide value 276.9 | Weak odour |
| 10 % ozonised sunflower oil, peroxide value 276.9 | No unpleasant odour |
| 30 % ozonised sunflower oil, peroxide value 276.9 | No unpleasant odour |

*Bacterial growth measurements*

Test liquid 1 was used for bacterial growth measurements: Sterile, synthetic urine to which a growth medium for microorganisms had been added. The synthetic urine contained monovalent and divalent cations and anions and urea and had been produced in accordance with the information in Geigy, Scientific Tables, vol. 2, 8th ed., 1981, page 53. The growth medium for the microorganisms is based on two common growth media, Hook and FSA medium for enterobacteria. The pH in this mixture was 6.6.

A homogenous mixture of fluffed pulp was prepared in the following way (Method 1)

Untreated and treated Weyerhauser pulp (NB416) was weighed in desired proportions and put in Braun multimixer, MX32. The pulp was mixed about 30 seconds.

Absorbent cores for testing were produced in the following way (Method 2):

Absorbent cores were prepared using a slightly modified sample former according to SCAN C 33:80. Fluffed pulp of the desired type(s) was weighed and a homogeneous mixture of the fluffed pulp(s) was introduced into a flow of air having a negative pressure of approximately 75 mbar, through a pipe having a diameter of 10 mm and being equipped at the bottom with a metal net. The fluff pulp was gathered on the metal net and thereafter constituted the absorbent specimen. The absorbent core was compressed to a bulk within the range of 6 to 12 $cm^3$/g.

Two different absorbent cores were produced; the reference core composed of 2.0g untreated Weyerhauser pulp (NB 416) and the test core composed of a mixture of 1.4g treated Weyerhauser pulp (NB 416), treated with oxidized sunflower oil, peroxide value 65.5 meq./kg, according to the method described under "Treatment of pulp with oils/fats" above (added amount was 30 weight% oil), and 1.0g of untreated Weyerhauser pulp (NB 416). The size of the absorbent cores was 5 cm in diameter.

The bacterial growth in the absorbent cores was measured in the following way (Method 3):

10 ml of test liquid 1 containing bacteria were added to a test core placed in a sterile jar (Nunc sputum/organ jars, 100 ml), and a lid was fitted on the jar. The jar was turned upside down and incubated in a warm cabinet at 35°C. After incubation for 0, 6 and 12 hours, the test cores were placed in a plastic bag with peptone water and the content was homogenized (agitated and worked up) in a stomacker for 3 minutes. The homogenate was diluted in dilution tubes with peptone water and a microbiological culture was spread on agar plates. Slanetz Bartley agar was used for E. faecalis, and Drigalski agar for E. coli and P. mirabilis. The specimens were incubated at 35°C for 1-2 days before the colonies were counted and the log CFU/ml calculated. Control tests were also carried out with absorbent cores

*Test results: Bacterial growth*

Bacteria were cultured in nutrient broth and diluted to the desired concentration of ca. Log 3.3 in test liquid 1 (method 3). Absorbent test cores were produced according to method 2. The bacterial growth was measured according to method 3.
The result is shown in Table 6, which clearly illustrates that the growth of all 3 test bacteria is considerably lower after 6 and 12 hours, compared with the reference core.

**Table 6**

| | 0h | | | 6h | | | 12h | | | pH | | |
|---|---|---|---|---|---|---|---|---|---|---|---|---|
| Sample | E.coli | P.mir. | E.faec. | E.coli | P.mir. | E.faec. | E.coli | P.mir. | E.faec. | 0h | 6h | 12h |
| Test 1 | 3.5 | 3.2 | 3.2 | <2 | <2 | <2 | <2 | <2 | <2 | 6.2 | 5.9 | 5.6 |
| Test 2 | 3.4 | 3.2 | 3.3 | <2 | <2 | <2 | <2 | <2 | <2 | 6.3 | 5.9 | 5.6 |
| **Test mv** | **3.5** | **3.2** | **3.3** | **<2** | **<2** | **<2** | **<2** | **<2** | **<2** | **6.2** | **5.9** | **5.6** |
| Ref. 1 | 3.5 | 3.2 | 3.2 | 6.1 | 5.0 | 6.3 | 9.7 | 8.8 | 9.1 | 6.6 | 6.6 | 8.9 |
| Ref. 2 | 3.4 | 3.2 | 3.3 | 6.5 | 5.1 | 6.2 | 8.8 | 7.8 | 8.1 | 6.6 | 6.6 | 8.6 |
| **Ref. mv** | **3.5** | **3.2** | **3.3** | **6.3** | **5.0** | **6.3** | **9.5** | **8.5** | **8.8** | **6.6** | **6.6** | **8.7** |

**Claims**

1.  An absorbent article such as a sanitary napkin, panty liner, diaper, pant diaper, adult incontinence guard, said article comprises an absorbent core (4) and a fluid impervious backsheet (3) *characterized in that* to said article has been added at least one oxidized lipid as an odour control substance, said lipid has been oxidized under controlled condition and has a peroxide value as measured by AOCS Official Method Cd 8-53 of at least 20 meq/kg.

2.  Absorbent article as claimed in claim 1, *characterized in that* the oxidized lipids have a peroxide value as measured by AOCS Official Method Cd 8-53 of at least 30 and preferably at least 40 meq/kg.

3.  Absorbent article as claimed in claim 1 or 2, *characterized in* that said absorbent core (4) comprises hydrophilic fibers treated with said oxidized lipids.

4.  Absorbent article as claimed in claim 3, *characterized in* that at least part of said hydrophilic fibers are cellulose fibers.

5.  Absorbent article as claimed in claim 3 or 4, *characterized in* that to said absorbent core (4) has been added at least 0.2% by weight of the oxidized lipids, calculated on the total weight of the hydrophilic fibers contained in the absorbent core.

6.  Absorbent article as claimed in claim 5, *characterized in* that to said absorbent core (4) has been added between 0.2 and 50% by weight, preferably between 0.5 and 40% by weight, more preferably between 1 and 35% by weight and most preferably between 3 and 30% by weight of the oxidized lipids, calculated on the total weight of the hydrophilic fibers contained in the absorbent core.

7. Absorbent article as claimed in any of the preceding claims, *characterized in* **that** the article further comprises a liquid-pervious topsheet (2) and/or one or more additional functional layers selected from: liquid receiving layers, liquid distribution layers.

8. Absorbent article as claimed in claim 7, *characterized in* **that** to the topsheet and/or to at least one of said additional functional layers has been added at least one oxidized lipid as an odour control substance.

9. Absorbent article as claimed in claim 8, *characterized in* **that** to said topsheet or additional functional layer has been added at least 0.2% by weight of the oxidized lipids, calculated on the total weight of said topsheet and/or additional functional layer.

10. Absorbent article as claimed in claim 9, *characterized in* **that** to said topsheet and/or additional functional layer has been added between 0.2 and 50% by weight, preferably between 0.5 and 40% by weight, more preferably between 1 and 35% by weight and most preferably between 3 and 30% by weight of the oxidized lipids, calculated on the total weight of the topsheet and/or additional functional layer.

11. Absorbent article as claimed in any of the preceding claims, said article having a longitudinal (y) and a transverse (x) direction, a pair of longitudinal edges (6,7) and a pair of transverse edges (8,9), *characterized in* **that** the odour control substance in form of oxidized lipids is located in higher amounts in areas close to the longitudinal and/or transverse edges of the article than in the central area of the article, intended as the fluid-receiving area.

12. Absorbent article as claimed in any of the preceding claims, said article comprising elastic means, for example leg elastics and/or waist elastics, *characterized in* **that** at least one oxidized lipid has been added to said elastic means.

13. Absorbent article as claimed in any of the preceding claims, *characterized in* **that** the lipids are fatty acids or derivatives thereof.

14. Absorbent article as claimed in claim 13, *characterized in* **that** the fatty acid derivatives are esters of fatty acids, especially triglycerides.

15. Absorbent article as claimed in claim 13 or 14, *characterized in* **that** at least part of the fatty acids and/or fatty acid derivatives are unsaturated.

16. Absorbent article as claimed in any of the preceding claims, *characterized in* **that** said oxidized lipids are oxidized by treatment with ozone.


**Patentansprüche**

1. Absorbierendes Erzeugnis, wie eine Damenbinde, Slipeinlage, Windel, Hosenwindel, Inkontinenzschutz für Erwachsene, wobei das Erzeugnis einen absorbierenden Kern (4) und eine flüssigkeitsundurchlässige Rückseitenlage (3) umfasst, **dadurch gekennzeichnet, dass** dem Erzeugnis mindestens ein oxidiertes Lipid als Geruchsreguliersubstanz hinzugefügt ist, worin das Lipid unter kontrollierten Bedingungen oxidiert wurde und einen Peroxidwert, wie durch "AOCS Official Method Cd 8-53" gemessen, von mindestens 20 mÄq./kg hat.

2. Absorbierendes Erzeugnis gemäß Anspruch 1, **dadurch gekennzeichnet, dass** die oxidierten Lipide Peroxidwerte, wie durch "AOCS Offical Method Cd 8-53" gemessen, von mindestens 30 und vorzugsweise mindestens 40 mÄq./kg haben.

3. Absorbierendes Erzeugnis gemäß Anspruch 1 oder 2, **dadurch gekennzeichnet, dass** der absorbierende Kern (4) hydrophile Fasern umfasst, die mit den oxidierten Lipiden behandelt sind.

4. Absorbierendes Erzeugnis gemäß Anspruch 3, **dadurch gekennzeichnet, dass** zumindest ein Teil der hydrophilen Fasern Zellulosefasern sind.

5. Absorbierendes Erzeugnis gemäß Anspruch 3 oder 4, **dadurch gekennzeichnet, dass** dem absorbierenden Kern (4) mindestens 0,2 Gew.% der oxidierten Lipide hinzugefügt wurden, berechnet auf Basis des Gesamtgewichts der hydrophilen Fasern, die im absorbierenden Kern enthalten sind.

6. Absorbierendes Erzeugnis gemäß Anspruch 5, **dadurch gekennzeichnet, dass** dem absorbierenden Kern (4) zwischen 0,2 und 50 Gew.%, vorzugsweise zwischen 0,5 und 40 Gew.% stärker bevorzugt zwischen 1 und 35 Gew.% und am stärksten bevorzugt zwischen 3 und 30 Gew.% der oxidierten Lipide hinzugefügt wurden, berechnet auf Basis des Gesamtgewichts der hydrophilen Fasern, die im absorbierenden Kern enthalten sind.

7. Absorbierendes Erzeugnis gemäß irgendeinem der vorhergehenden Ansprüche, **dadurch gekennzeichnet, dass** das Erzeugnis weiterhin eine flüssigkeitsdurchlässige obere Lage (2) und/oder ein oder mehrere zusätzliche funktionelle Schichten, ausgewählt aus flüssigkeitsaufnehmenden Schichten und flüssigkeitsverteilenden Schichten, umfasst.

8. Absorbierendes Erzeugnis gemäß Anspruch 7, **dadurch gekennzeichnet, dass** der oberen Lage und/oder mindestens einer der zusätzlichen funktionellen Schichten mindestens ein oxidiertes Lipid als Geruchsreguliersubstanz hinzugefügt wurde.

9. Absorbierendes Erzeugnis gemäß Anspruch 8, **dadurch gekennzeichnet, dass** der oberen Lage oder der funktionellen Schicht mindestens 0,2 Gew.% der oxidierten Lipide hinzugefügt wurden, berechnet auf Basis des Gesamtgewichts der oberen Lage und/oder zusätzlichen funktionalen Schicht.

10. Absorbierendes Erzeugnis gemäß Anspruch 9, **dadurch gekennzeichnet, dass** der oberen Lage und/oder zusätzlichen funktionalen Schicht zwischen 0,2 und 50 Gew.%, vorzugsweise zwischen 0,5 und 40 Gew.%, stärker bevorzugt zwischen 1 und 35 Gew.% und am stärksten bevorzugt zwischen 3 und 30 Gew.% der oxidierten Lipide, hinzugefügt wurden, berechnet auf Basis des Gesamtgewichts der oberen Lage und/oder zusätzlichen funktionalen Schicht.

11. Absorbierendes Erzeugnis gemäß irgendeinem der vorhergehenden Ansprüche, wobei das Erzeugnis eine Längs-(y) und eine Quer-(x)Richtung sowie ein Paar von Längskanten (6, 7) und ein Paar von Querkanten (8, 9) aufweist, **dadurch gekennzeichnet, dass** die Geruchsreguliersubstanz in Form oxidierter Lipide in Bereichen nahe der Längs- und/oder Querkanten des Erzeugnisses in höheren Mengen lokalisiert ist als im Zentralbereich des Erzeugnisses, der als flüssigkeitsaufnehmende Fläche vorgesehen ist.

12. Absorbierendes Erzeugnis gemäß irgendeinem der vorhergehenden Ansprüche, wobei das Erzeugnis elastische Mittel umfasst, beispielsweise elastische Elemente für die Beine und/oder elastische Elemente für die Taille, **dadurch gekennzeichnet, dass** mindestens ein oxidiertes Lipid zu den elastischen Mitteln hinzugefügt ist.

13. Absorbierendes Erzeugnis gemäß irgendeinem der vorhergehenden Ansprüche, **dadurch gekennzeichnet, dass** die Lipide Fettsäuren oder Derivate davon sind.

14. Absorbierendes Erzeugnis gemäß Anspruch 13, **dadurch gekennzeichnet, dass** die Fettsäurederivate Ester von Fettsäuren, insbesondere Triglyceride sind.

15. Absorbierendes Erzeugnis gemäß Anspruch 13 oder 14, **dadurch gekennzeichnet, dass** zumindest ein Teil der Fettsäuren und/oder Fettsäurederivate ungesättigt ist.

16. Absorbierendes Erzeugnis gemäß irgendeinem der vorhergehenden Ansprüche, **dadurch gekennzeichnet, dass** die oxidierten Lipide durch Behandlung mit Ozon oxidiert worden sind.

**Revendications**

1. Article absorbant tel qu'une serviette hygiénique, un protège-slip, une couche, une couche-culotte, une protection contre l'incontinence pour adultes, ledit article comprenant un noyau absorbant (4) et une feuille arrière (3) imperméable aux liquides *caractérisé en ce qu*'audit article a été ajouté au moins un liquide oxydé tel qu'une substance de maîtrise des odeurs, ledit lipide ayant été oxydé dans des conditions contrôlées et étant doté d'une valeur de peroxydation d'au moins 20 meq/kg, mesurée selon la Méthode Officielle AOCS, Cd 8-53.

**2.** Article absorbant selon la revendication 1 *caractérisé en ce que* les lipides oxydés sont dotés d'une valeur de peroxydation d'au moins 30 meq/kg et de préférence d'au moins 40 meq/kg, mesurée selon la Méthode Officielle AOCS, Cd 8-53.

**3.** Article absorbant selon la revendication 1 ou 2 *caractérisé en ce que* ledit noyau absorbant (4) comprend des fibres hydrophiles traitées avec lesdits lipides oxydés.

**4.** Article absorbant selon la revendication 3 *caractérisé en ce qu'*au moins une partie desdites fibres hydrophiles sont des fibres de cellulose.

**5.** Article absorbant selon la revendication 3 ou 4 *caractérisé en ce qu'*audit noyau absorbant (4) a été ajouté au moins 0,2 % en poids de lipides oxydés, calculé sur le poids total des fibres hydrophiles contenues dans le noyau absorbant.

**6.** Article absorbant selon la revendication 5 *caractérisé en ce qu'*audit noyau absorbant (4) a été ajouté 0,2 à 50 % en poids, de préférence 0,5 à 40 % en poids, plus préférablement 1 à 35 % en poids et le plus préférablement 3 à 30 % en poids des lipides oxydés, calculé sur le poids total des fibres hydrophiles contenues dans le noyau absorbant.

**7.** Article absorbant selon l'une quelconque des revendications qui précèdent *caractérisé en ce que* l'article comporte en outre une feuille supérieure (2) perméable aux liquides et/ou une ou plusieurs couches fonctionnelles supplémentaires choisies parmi les couches de réception de liquides et les couches de répartition de liquides.

**8.** Article absorbant selon la revendication 7 *caractérisé en ce qu'*à la feuille supérieure et/ou à au moins une desdites couches fonctionnelles supplémentaires, a été ajouté au moins un lipide oxydé tel qu'une substance de maîtrise des odeurs.

**9.** Article absorbant selon la revendication 8 *caractérisé en ce qu'*à ladite feuille supérieure ou à une couche fonctionnelle supplémentaire a été ajouté au moins 0, 2 % en poids de lipides oxydés, calculé sur le poids total de ladite couche supérieure et/ou sur celui de la couche fonctionnelle supplémentaire.

**10.** Article absorbant selon la revendication 9 *caractérisé en ce qu'*à ladite couche supérieure et/ou à la couche fonctionnelle supplémentaire a été ajouté 0,2 à 50 % en poids, de préférence 0,5 à 40 % en poids, plus préférablement 1 à 35 % en poids, et le plus préférablement 3 à 30 % en poids de lipides oxydés, calculé sur le poids total de la feuille supérieure et/ou sur celui de la couche fonctionnelle supplémentaire.

**11.** Article absorbant selon l'une quelconque des revendications qui précèdent, ledit article ayant une direction longitudinale (y) et une direction transversale (x), une paire de bords longitudinaux (6, 7) et une paire de bords transversaux (8, 9) *caractérisé en ce que* la substance de maîtrise des odeurs sous forme de lipides oxydés est située dans les zones proches des bords longitudinaux et/ou des bords transversaux de l'article en des quantités plus importantes que dans la zone centrale de l'article, destinées à être une zone de réception de fluides.

**12.** Article absorbant selon l'une quelconque des revendications qui précèdent, ledit article comprenant des moyens élastiques, par exemple des élastiques de jambe et/ou des élastiques de taille, *caractérisé en ce qu'*au moins un lipide oxydé a été ajouté audit moyen élastique.

**13.** Article absorbant selon l'une quelconque des revendications qui précèdent *caractérisé en ce que* les lipides sont des acides gras ou des dérivés de ceux-ci.

**14.** Article absorbant selon la revendication 13 *caractérisé en ce que* les dérivés d'acides gras sont des esters d'acides gras, en particulier des triglycérides.

**15.** Article absorbant selon la revendication 13 ou 14 *caractérisé en ce qu'au* moins une partie des acides gras et/ou des dérivés d'acides gras sont insaturés.

**16.** Article absorbant selon l'une quelconque des revendications qui précèdent *caractérisé en ce que* lesdits lipides oxydés sont oxydés par un traitement à l'ozone.

1

8

5

6

7

II

II

y

x

*Fig.1*

4

2

5

5

10

11

3

*Fig.2*

9

## REFERENCES CITED IN THE DESCRIPTION

**Patent documents cited in the description**

- US 6147028 A
- US 6479150 B
- GB 1282889 A
- WO 2007067111 A
- US 2356062 A
- EP 1192955 A
- EP 1035818 A
- WO 2007073270 A

**Non-patent literature cited in the description**

- *Bailey's Industrial Oil and Fat products,* 1979, vol. 1